(19)

Europäisches Patentamt
European Patent Office
Office européen des brevets

(11) **EP 1 961 413 A1**

(12) **EUROPEAN PATENT APPLICATION**
published in accordance with Art. 158(3) EPC

(43) Date of publication:
27.08.2008 Bulletin 2008/35

(51) Int Cl.:
A61K 9/20 (2006.01)   A61K 31/4545 (2006.01)
A61K 47/10 (2006.01)   A61K 47/18 (2006.01)
A61K 47/22 (2006.01)   A61K 47/26 (2006.01)
A61K 47/34 (2006.01)   A61K 47/36 (2006.01)
A61K 47/38 (2006.01)   A61K 47/42 (2006.01)
A61K 47/44 (2006.01)   A61K 47/46 (2006.01)
A61P 37/08 (2006.01)

(21) Application number: 06768345.8

(22) Date of filing: 21.07.2006

(86) International application number:
PCT/JP2006/314467

(87) International publication number:
WO 2007/011018 (25.01.2007 Gazette 2007/04)

(84) Designated Contracting States:
AT BE BG CH CY CZ DE DK EE ES FI FR GB GR HU IE IS IT LI LT LU LV MC NL PL PT RO SE SI SK TR

(30) Priority: 22.07.2005  JP 2005212836

(71) Applicant: Mitsubishi Tanabe Pharma Corporation
Chuo-ku
Osaka-shi
Osaka 541-8505 (JP)

(72) Inventors:
• UEMURA, Katsuji
c/o MITSUBISHI TANABE PHARMA CORPORATION
Osaka 541-8505 (JP)

• SUGIMOTO, Masaaki
c/o MITSUBISHI TANABE PHARMA CORPORATION
Osaka 541-8505 (JP)
• TAKATSUKA, Shinya
c/o MITSUBISHI TANABE PHARMA CORPORATION
Osaka 541-8505 (JP)
• KOMORI, Toshinobu
c/o MITSUBISHI TANABE PHARMA CORPORATION
Osaka 541-8505 (JP)

(74) Representative: HOFFMANN EITLE
Patent- und Rechtsanwälte
Arabellastrasse 4
81925 München (DE)

(54) **RAPIDLY DISINTEGRATABLE ORAL TABLET**

(57)    The present invention provides a method of suppressing the bitter taste of a drug when a rapidly disintegrating tablet in an oral cavity is produced.

When a rapidly disintegrating tablet in an oral cavity is produced, the bitter taste of a drug is suppressed by utilizing (a) a granule which contains a drug having a bitter taste and an excipient and in which the bitter taste has not been suppressed and (b) a granule containing a water soluble saccharide.

[Fig. 1]

EP 1 961 413 A1

**Description**

TECHNICAL FIELD

The present invention relates to a method of suppressing a bitter taste of a drug in the production of a rapidly disintegrating tablet in an oral cavity and a method of producing a rapidly disintegrating tablet in an oral cavity in which a bitter taste has been suppressed.

Moreover, the present invention relates to a rapidly disintegrating tablet in an oral cavity containing bepotastine besilate, which utilizes the method of suppressing a bitter taste of the present invention and a production method therefor.

BACKGROUND ART

Recently, as a pharmaceutical preparation which is easily taken by young children, elderly persons, and patients who suffer from severe diseases whose swallowing force is weak, and moreover, as its fast-acting property is expected, a rapidly disintegrating tablet in an oral cavity has been used. As a method of producing a rapidly disintegrating tablet in an oral cavity by compression-molding, for example, the following methods are known:

(1) a method in which after a drug formulation component comprising a drug and an alcohol soluble binder is molded in a low density, it is first wetted with alcohol, followed by removal of the alcohol (Patent Document 1),

(2) a method in which a mixture containing a drug, a saccharide and moisture is made into a tablet (Patent Document 2),

(3) a method in which after a mixture of a drug, a water soluble binder and a water soluble excipient is made into a tablet, it is wetted with moisture vapor, followed by drying (Patent Document 3),

(4) a method in which a kneaded product containing a water soluble additive, a drug and water is compression-molded, and then dried, followed by glazing (Patent Document 4).

(5) a method in which after a drug and a saccharide whose compactibility is low are granulated using a saccharide whose compactibility is high, the granulated product is compression-molded (Patent Document 5), and

(6) a method in which after a drug, a water soluble excipient and a non-crystalline saccharide are compression-molded, aging is performed therefor (Patent Document 6).

Moreover, at the time when a rapidly disintegrating tablet in an oral cavity containing a drug that has an uncomfortable taste such as a bitter taste and an excipient is produced, a variety of methods in which the taste of a drug is suppressed have been studied, and the following methods are known, for example:

(7) a method of producing a rapidly disintegrating tablet in an oral cavity in which a particle obtained by spraying a suspension containing a drug having a bitter taste and a carrier for pharmaceutical preparation into a rotary disc type spray drier is mixed with saccharides, followed by compressing (Patent Document 7),

(8) a solid medicine composition of orally dissolving or chewable type for oral administration in which menthol in the range of 0.1 to 2.25 w/w% of the composition is blended with a drug having a bitter taste (Patent Document 8), and

(9) a solid pharmaceutical preparation for oral administration in which menthol and one or two or more kinds of sweeteners selected from the group consisting of stevia extract, aspartame, glycyrrhizinic acid, saccharin and sucralose are blended with a component having an uncomfortable taste (Patent Document 9).

Furthermore, a method of producing a rapidly disintegrating tablet in an oral cavity whose blending amount of an easily soluble drug is 5% by weight or more which comprises, for the purpose of reducing the delay of disintegration of the rapidly disintegrating tablet in an oral cavity containing the easily soluble drug, mixing a soluble particle having an average particle diameter of 100 $\mu$m or more which contains the easily soluble drug in a high concentration and a rapidly disintegrating component and compressing, has been also proposed (Patent Document 10).

| | |
|---|---|
| [Patent Document 1] | Japanese Patent Application Laid-Open (JP-A) No. Hei 10-298061 |
| [Patent Document 2] | JP-A No. Hei 5-271054 |
| [Patent Document 3] | JP-A No. Hei 8-291051 |
| [Patent Document 4] | WO93/15724 Pamphlet |
| [Patent Document 5] | WO95/20380 Pamphlet |
| [Patent Document 6] | JP-A No. Hei 11-12161 |
| [Patent Document 7] | WO02/02083 Pamphlet |
| [Patent Document 8] | JP-A No. 2000-95707 |

(continued)

| [Patent Document 9] | JP-A No. 2000-159691 |
| [Patent Document 10] | JP-A No. 2001-253818 |

## DISCLOSURE OF INVENTION

## PROBLEMS TO BE SOLVED BY THE INVENTION

The present invention relates to a method of suppressing effectively a bitter taste of a drug at the time when it is disintegrated in an oral cavity, the method comprising using a granule which contains a drug having a bitter taste and an excipient and in which the bitter taste of the drug has not been suppressed, and a granule containing a water soluble saccharide, and further a method of producing a rapidly disintegrating tablet in an oral cavity in which a bitter taste has been suppressed by utilizing the suppressing method.

Furthermore, the present invention relates to a rapidly disintegrating tablet in an oral cavity containing bepotastine besilate, which utilizes this method of suppressing a bitter taste, and a production method therefor.

## MEANS TO SOLVE THE PROBLEM

The present invention has been completed based on the novel finding that at the time when a rapidly disintegrating tablet in an oral cavity is produced, a bitter taste of a drug in the tablet can be unexpectedly and effectively suppressed by utilizing both of a granule containing a drug having a bitter taste and an excipient and a granule containing a water soluble saccharide and by taking no measures for suppressing the bitter taste of the drug to the granules containing the drug having the bitter taste and the excipient.

Throughout this specification, "a granule in which a bitter taste has not been suppressed" means the granule from which the drug release in an oral cavity has not been suppressed (e.g., the granule from which the drug dissolution in an oral cavity has not been suppressed by controlling the drug dissolution).

## BRIEF DESCRIPTION OF THE DRAWINGS

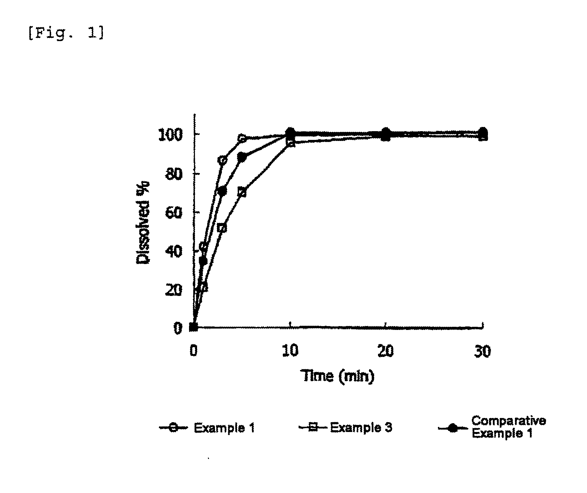

[Fig.1] Fig. 1 is a graphical representation showing the results of dissolution test of bepotastine besilate in purified water in accordance with the Japanese Pharmacopoeia Fourteen Edition , dissolution test method, paddle method (number of paddle rotations; 50 rpm; 37°C) for the rapidly disintegrating tablets in an oral cavity of Examples 1, 3 and Comparative Example 1.

## BEST MODE FOR CARRYING OUT THE INVENTION

The present invention relates to a method of suppressing a bitter taste of a drug which comprises, when a rapidly disintegrating tablet in an oral cavity is produced, utilizing

(a) a granule which contains a drug having a bitter taste and an excipient and in which the bitter taste has not been suppressed and (b) a granule containing a water soluble saccharide.

The present invention relates to a method of producing a rapidly disintegrating tablet in an oral cavity comprising the following steps:

(1) a step of mixing (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent,
(2) a step of compression-molding the mixture of (1), and
(3) a step of treating the compression-molded product of (2) with an alcohol based solvent.

Furthermore, the present invention relates to a rapidly disintegrating tablet in an oral cavity containing the following three components, which utilizes a method of suppressing a bitter taste according to the present invention:

(a) a granule which contains bepotastine besilate and an excipient and in which a bitter taste has not been suppressed;

(b) a granule containing a water soluble saccharide which is insoluble in an alcohol based solvent and a binder which is soluble in water and in an alcohol based solvent; and

(c) a cool flavoring ingredient, a sweetener and a lubricant.

Furthermore, the present invention relates to a method of producing a rapidly disintegrating tablet in an oral cavity comprising the following steps, which utilizes a method of suppressing a bitter taste according to the present invention.

(1) a step of mixing (a) a granule which contains bepotastine besilate and an excipient and in which a bitter taste has not been suppressed, (b) a granule containing a water soluble saccharide which is insoluble in an alcohol based solvent and a binder which is soluble in water and in an alcohol based solvent, and (c) a cool flavoring ingredient, a sweetener and a lubricant,

(2) a step of compression-molding the mixture of (1), and

(3) a step of treating the compression-molded product of (2) with an alcohol based solvent.

Furthermore, in the method of suppressing a bitter taste of a drug according to the present invention, it is preferable that (c) a cool flavoring ingredient and a sweetener are used, wherein (A) (c) the cool flavoring ingredient and the sweetener may be blended into one or both of (a) the granule which contains a drug having a bitter taste and an excipient and in which the bitter taste has not been suppressed and (b) the granule containing a water soluble saccharide, or (B) (c) the cool flavoring ingredient and the sweetener may be blended as a component in addition to (a) a granule which contains a drug having a bitter taste and an excipient and in which the bitter taste has not been suppressed and (b) a granule containing a water soluble saccharide.

It is preferable that both of (a) the granule which contains a drug having a bitter taste and an excipient and in which the bitter taste has not been suppressed and (b) the granule containing a water soluble saccharide [in any case where (c) the cool flavoring ingredient and the sweetener are blended or not blended ] are in the range of 50 to 250 $\mu$m for the average particle diameter so as not to generate surface roughness feeling after the disintegration in an oral cavity, and it is more preferable that they are made in the range of 100 to 200 $\mu$m for the average particle diameter.

It is preferable that (c) a cool flavoring ingredient and a sweetener are blended in a form of a powder or in a form in which these components have been preblended with excipients when (c) the cool flavoring ingredient and a sweetener are blended as a component in addition to (a) a granule which contains a drug having a bitter taste and an excipient and in which the bitter taste has not been suppressed and (b) a granule containing a water soluble saccharide. The method of suppressing a bitter taste of a drug according to the present invention is applicable to a granular preparation by mixing (a) a granule which contains a drug having a bitter taste and an excipient and in which the bitter taste has not been suppressed and (b) a granule containing a water soluble saccharide, if necessary, together with a cool flavoring ingredient and a sweetener, and is also applicable to a rapidly disintegrating tablet in an oral cavity by mixing (a) a granule which contains a drug having a bitter taste and an excipient and in which the bitter taste has not been suppressed and (b) a granule containing a water soluble saccharide, if necessary, together with a cool flavoring ingredient and a sweetener, followed by molding of the mixture into a tablet according to the conventionally known methods.

Moreover, when the method of suppressing a bitter taste of a drug according to the present invention is applied to a rapidly disintegrating tablet in an oral cavity, (a) a granule which contains a drug having a bitter taste and an excipient and in which the bitter taste has not been suppressed and (b) a granule containing a water soluble saccharide can be contained respectively, in (a) the range of 2 to 40 w/w% of the rapidly disintegrating tablet in an oral cavity [the drug having a bitter taste is contained in the range of 1 to 35 w/w% of the rapidly disintegrating tablet in an oral cavity] and in (b) the range of 40 to 97 w/w% of the rapidly disintegrating tablet in an oral cavity [the water soluble saccharide is contained in the range of 20 to 96 w/w% of the rapidly disintegrating tablet in an oral cavity]. And it is preferable that, the above-mentioned granules (a) and (b) are contained respectively in (a) the range of 5 to 30 w/w% of the rapidly disintegrating tablet in an oral cavity [the drug having a bitter taste is contained in the range of 1 to 15 w/w% of the rapidly disintegrating tablet in an oral cavity] and in (b) the range of 60 to 95 w/w% [the water soluble saccharide is contained in the range of 55 to 90 w/w% of the rapidly disintegrating tablet in an oral cavity].

Moreover, when (c) the cool flavoring ingredient and the sweetener are used, the cool flavoring ingredient and the sweetener can be contained in the range of 0.1 to 1 w/w% of the rapidly disintegrating tablet in an oral cavity and in the range of 0.2 to 5 w/w% of the rapidly disintegrating tablet in an oral cavity, respectively, and it is preferable that the cool flavoring ingredient and the sweetener are contained in the range of 0.2 to 0.7 w/w% of the rapidly disintegrating tablet in an oral cavity and in the range of 0.5 to 3 w/w% of the rapidly disintegrating tablet in an oral cavity, respectively.

In the method of producing a rapidly disintegrating tablet in an oral cavity according to the present invention, furthermore, it is preferable that (c) a cool flavoring ingredient, a sweetener and a lubricant are used, wherein

(A) (c) the cool flavoring ingredient and the sweetener may be blended to one or both of (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent, and the lubricant may be mixed as a component in addition to (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent, or

(B) (c) the cool flavoring ingredient, the sweetener and the lubricant may be mixed as a component in addition to (a) a granule which contains a drug having a bitter taste and an excipient and in which the bitter taste has not been suppressed, and (b) a granule containing a water soluble saccharide which is insoluble in an alcohol based solvent, and a binder which is soluble in water and in an alcohol based solvent.

It is preferable that both of (a) the granule which contains a drug having a bitter taste and an excipient and in which the bitter taste has not been suppressed and (b) the granule containing a water soluble saccharide which is insoluble in an alcohol based solvent and a binder which is soluble in water and in an alcohol based solvent [in any case where (c) the cool flavoring ingredient and a sweetener are blended or not blended] are in the range of 50 to 250 $\mu$m for the average particle diameter so as not to generate surface roughness feeling after the disintegration in an oral cavity, and it is more preferable that it is in the range of 100 to 200 $\mu$m for the average particle diameter.

When (c) a cool flavoring ingredient, a sweetener and a lubricant are mixed as a component in addition to (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent, it is preferable that the lubricant is mixed as a powder, and the cool flavoring ingredient and the sweetener are blended in the form of a powder or in the form in which these components are preblended with excipients.

Moreover, (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent can be contained respectively, in (a) the range of 2 to 40 w/w% of the rapidly disintegrating tablet in an oral cavity [the drug having a bitter taste is contained in the range of 1 to 35 w/w% of the rapidly disintegrating tablet in an oral cavity] and in (b) the range of 40 to 97 w/w% of the rapidly disintegrating tablet in an oral cavity [the water soluble saccharide which is insoluble in an alcohol based solvent is contained in the range of 20 to 96 w/w% of the rapidly disintegrating tablet in an oral cavity, and the binder which is soluble in water and in an alcohol based solvent is contained in the range of 0.5 to 10 w/w% of the rapidly disintegrating tablet in an oral cavity]. And it is preferable that the above-mentioned granules (a) and (b) are contained respectively in (a) the range of 5 to 30 w/w% of the rapidly disintegrating tablet in an oral cavity [the drug having a bitter taste is contained in the range of 1 to 20 w/w% of the rapidly disintegrating tablet in an oral cavity] and in (b) the range of 60 to 95 w/w% of the rapidly disintegrating tablet in an oral cavity [the water soluble saccharide which is insoluble in an alcohol based solvent is contained in the range of 55 to 90 w/w% of the rapidly disintegrating tablet in an oral cavity, and the binder which is soluble in water and in an alcohol based solvent is contained in the range of 1 to 5 w/w% of the rapidly disintegrating tablet in an oral cavity].

Moreover, when (c) a cool flavoring ingredient, a sweetener and a lubricant are used, the cool flavoring ingredient, the sweetener, and the lubricant can be contained respectively, in the range of 0.1 to 1 w/w% of the rapidly disintegrating tablet in an oral cavity, in the range of 0.2 to 5 w/w% of the rapidly disintegrating tablet in an oral cavity, and in the range of 0.2 to 2 w/w% of the rapidly disintegrating tablet in an oral cavity. And it is preferable that the cool flavoring ingredient, the sweetener, and the lubricant are contained respectively in the range of 0.2 to 0.7 w/w% of the rapidly disintegrating tablet in an oral cavity, in the range of 0.5 to 3 w/w% of the rapidly disintegrating tablet in an oral cavity, and in the range of 0.4 to 1.5 w/w% of the rapidly disintegrating tablet in an oral cavity.

Furthermore, in the rapidly disintegrating tablet in an oral cavity containing bepotastine besilate according to the present invention and a producing method therefor, while bepotastine besilate is used as a drug having a bitter taste, the respective constituting components, the average particle diameter of the respective constituting components and the content of the constituting components are the same as those of the method of producing a rapidly disintegrating tablet in an oral cavity according to the present invention.

As a drug having a bitter taste in the present invention, the following drugs can be listed.

(1) Antipyretic, analgesic and antiphlogistic (for example, diclofenac sodium, ketoprofen, ibprofen, isopropylantipyrine, acetoaminophenone, flufenamic acid, etodolac, epirizole, piroxicam and the like);
(2) Steroidal anti-inflammatory drug (for example, predonizolone);
(3) Antiulcer agent (for example, ecabet sodium, cimetidine, ranitidine hydrochloride, famotidine and the like);
(4) Coronary vasodilator (for example, diltiazem hydrochloride, dipyridamole and the like);
(5) Peripheral vasodilator (for example, todralazine hydrochloride, niceritrol and the like);
(6) Antibiotic drug (for example, bacampicillin hydrochloride, clarithromycin, chloramphenicol, erithromycin and the like);

(7) Synthesized antibacterial drug (for example, enoxacin and the like);

(8) Antiviral drug (for example, aciclovir and the like);

(9) Antispasmodic drug (for example, propantheline bromide, N-methylscopolamine methylsulfate and the like);

(10) Antitussive drug (for example, methylephedrine hydrochloride, dextromethorphan hydrobromide, dimemorfan phosphate and the like);

(11) Expectorant (for example, ethyl cysteine hydrochloride and the like);

(12) Bronchodilator (for example, theophylline, aminophylline, diprophylline and the like);

(13) Cardiotonic (for example, docarpamine, caffeine and the like);

(14) Diuretic (for example, acetazolamide, hydrochlorothiazide, azosemide and the like);

(15) Muscle relaxant (for example, methocarbamol, pridinol mesylate and the like);

(16) Brain metabolism improving drug (for example, meclofenoxate hydrochloride, calcium hopantenate and the like);

(17) Minor tranquilizer (for example, diazepam, chlordiazepoxide and the like);

(18) Major tranquilizer (for example, chlorpromazine and the like);

(19) Beta-blocker (for example, propranolol hydrochloride, alprenolol hydrochloride and the like);

(20) Antiarrythmic drug (for example, quinidine sulfate and the like);

(21) Arthrifuge drug (for example, probenecid, bucolome and the like);

(22) Anticoagulant drug (for example, ticlopidine hydrochloride and the like);

(23) Drug for migraine (for example, caffeine anhydride, ergotamine tartrate and the like);

(24) Antiepileptic drug (for example, sodium valproate, carbamazepine and the like);

(25) Antiallergic drug (for example, chlorpheniramine maleate, mequitazine, diphenhydramine, bepotastine besilate and the like);

(26) Antiemetic drug (for example, betahistine mesylate, trimebutine maleate and the like);

(27) Antihypertensive drug (for example, indapamide, alacepril and the like);

(28) Drug for hyperlipidemia (for example, sodium pravastatin and the like);

(29) Sympathomimetic drug (for example, ethylefrine hydrochloride and the like);

(30) Alzheimer dementia therapeutic drug (for example, Donepezil hydrochloride and the like);

(31) Oral antitumor drug (for example, tegafur and the like);

(32) Alkaloidal narcotic drug (for example, codeine and the like);

(33) Vitamin (for example, fursulthiamin hydrochloride, thiamin nitrate, pyridoxine hydrochloride, calcium ascorbate and the like);

(34) Drug for increased frequency of micturition (for example, flavoxate hydrochloride and the like);

(35) Angiotensin conversion enzyme inhibition drug (for example, alacepril and the like);

(36) Drug for erectile dysfunction (for example, sildenafil citrate, vardenafil hydrochloride, avanafil and the like).

A water soluble saccharide refers to a saccharide which dissolves in an amount of 20 mg or more in 1 mL of water (25°C). As a water soluble saccharide, one or two or more kinds selected from mannitol, xylitol, erythritol, maltitol, sorbitol, lactose, sucrose, maltose and trehalose can be mentioned.

A water soluble saccharide which is insoluble in an alcohol based solvent refers to a saccharide which dissolves in an amount of less than 1 mg in 1 mL of an alcohol based solvent (25°C) among the water soluble saccharides. Here, as an alcohol based solvent, methanol, ethanol, n-propanol, i-propanol, n-butanol, 2-methoxyethanol (methyl cellosolve; produced by Katayama Chemical Industries, Co., Ltd.) and the like can be mentioned. It is preferable that alcohol having 1 to 4 carbon atoms such as methanol, ethanol, n-propanol, i-propanol and the like is used, and it is particularly preferable that ethanol is used.

As a water soluble saccharide which is insoluble in an alcohol based solvent, for example, one or two or more kinds selected from mannitol, xylitol, erythritol, maltitol, sorbitol, lactose, sucrose, maltose and trehalose can be mentioned.

Among these saccharides, endothermically soluble saccharides are preferable from the viewpoint that cool feeling is given at the time when it is dissolved in an oral cavity. It is preferable that a saccharide whose endothermic amount is 15 cal/g or more is used, it is particularly preferable that a saccharide whose endothermic amount is in the range of 20 to 50 cal/g is used. For example, mannitol, xylitol and erythritol can be mentioned, and it is particularly preferable that mannitol is used.

As an excipient, a water soluble saccharide, an organic excipient (for example, corn starch, potato starch, sodium carboxymethyl starch, partially alphanized starch, caboxymethyl cellulose calcium, carboxymethyl cellulose, low-substituted hydroxypropyl cellulose, crosslinking carboxymethyl celullose sodium, crosslinking polyvinyl pyrrolidone, crystalline celullose), an inorganic excipient (for example, calcium carbonate, calcium citrate, calcium phosphate, calcium hydrogenphosphate, calcium silicate, magnesium metasilicate aluminate) can be mentioned. It is preferable that a water soluble saccharide and an organic excipient are used, and it is particularly preferable that one or two kind(s) selected from mannitol and crystalline cellulose is/are used.

A binder which is soluble in water and in an alcohol based solvent refers to a binder which dissolves in an amount of 20 mg or more in both 1 mL of water (25°C) and in 1 mL of the above-mentioned alcohol based solvent (25°C). It is preferable that a polymer binder is used, and for example, hydroxypropyl celullose, and polyvinyl pyrrolidone can be mentioned. It is particularly preferable that polyvinyl pyrrolidone is used.

A cool flavoring ingredient refers to a component giving a cool feeling in an oral cavity with a small amount, and it includes natural and artificial components. For example, one or two or more kinds selected from menthol, cinerole, camphor, mentha oil, mint oil (peppermint oil, spearmint oil), mint powder, orange oil, fennel oil, cinnamon oil, clove oil, turpentine oil, eucalyptus oil and the like can be mentioned, and it is preferable that menthol, mentha oil and the like are used.

A sweetener provides a sweetened feeling of 150 or more times of white sugar within an oral cavity, and it includes a natural and artificial component. As a sweetener, for example, one or two or more kinds selected from an artificial sweetener such as saccharine, saccharine sodium, aspartame (1-methyl N-L-a-aspartyl-L-phenylalanine; produced by Ajinomoto, Co., Inc.), acesulfame potassium, sucralose and the like, and a natural sweetener such as stevia, sormatin and the like can be mentioned, and aspartame is preferably used.

As a lubricant, conventional lubricants can be used depending on the formulation. For example, one or two or more selected from a group consisting of talc, light anhydrous silicic acid, hydrated silicon dioxide, alkali earth metal stearate (for example, magnesium stearate, calcium stearate), sucrose higher fatty acid ester (for example, sucrose stearic acid ester, sucrose behenic acid ester), glycerin higher fatty acid ester (for example, glycerin behenic acid ester) can be used. It is preferable that one or two kind(s) selected from alkali earth metal stearates is/are used.

Moreover, in a granule which contains a drug having a bitter taste and an excipient and in which the bitter taste has not been suppressed, a binder (for example, polyethylene glycol, polyvinyl alcohol, polyvinyl pyrrolidone, hydroxypropyl celullose, hydroxypropylmethyl celullose, saccharose, dextrin), solubilizing agent (for example, cyclodextrin, arginine, lysine, trisaminomethane) as well as the above-mentioned lubricant, the above-mentioned cool flavoring ingredient, the above-mentioned sweetener and the like, may be contained in addition to the drug and the excipient depending on the formulation.

Moreover, in a granule containing a water soluble saccharide, the above-mentioned organic excipient, the above-mentioned inorganic excipient, the above-mentioned binder, the above-mentioned lubricant, the above-mentioned cool flavoring ingredient, the above-mentioned sweetener and the like, may be contained in addition to the water soluble saccharides depending on the formulation.

A granule containing a water soluble saccharide which is insoluble in an alcohol based solvent and a binder which is soluble in water and in an alcohol based solvent, is the same as a granule containing a water soluble saccharide, except for always containing a binder that is soluble in water and in an alcohol based solvent.

A cool flavoring ingredient and a sweetener may be in a form of a powder per se, or in a form in which these components have been preblended with other excipients or granules (for example, a granule containing microcrystalline celullose, light-anhydrous silicic acid, hydrated silicon dioxide, water soluble saccharide). Moreover, a coloring agent (for example, food dye red color No. 2 and No. 3, food dye yellow color No. 4 and No. 5, food dye green color No. 3, food dye blue color No. 1 and No. 2, aluminum lakes thereof, iron sesquioxide, yellow iron sesquioxide), correctives (for example, sodium chloride, sodium citrate, sodium glutamate, sodium bicarbonate), a solubilizing agent (for example, cyclodextrin, arginine, lysine, trisaminomethane) and the like may be added.

A rapidly disintegrating tablet in an oral cavity (including a rapidly disintegrating tablet in an oral cavity containing bepotastine besilate) obtained according to the producing method of the present invention has a degree of hardness of 20N or more, preferably 25N or more and more preferably 30 to 80N, and the porosity calculated by the following equation is 20 to 50%, and preferably 22 to 40%.

$$\text{Porosity (\%)} = (V \times \rho - M) / (V \times \rho) \times 100$$

[In the equation, V represents a volume (mL) of a rapidly disintegrating tablet in an oral cavity, and $\rho$ represents a density (g/mL) of the portion except for the pores of the rapidly disintegrating tablet in an oral cavity, and M represents a weight (g) of the rapidly disintegrating table in an oral cavity].

Moreover, as for the disintegration time of a rapidly disintegrating tablet in an oral cavity, it is within 60 seconds in an oral cavity, preferably 5 to 45 seconds, and more preferably within 10 to 30 seconds. It is within 200 seconds in the disintegrating test method stipulated in the Japanese Pharmacopoeia Fourteenth Edition, (up and down movement: 29 to 32 reciprocation/min; 37°C; water), preferably within 150 seconds, and more preferably 30 to 100 seconds.

A rapidly disintegrating tablet in an oral cavity using the method of suppressing a bitter taste according to the present invention can be produced in accordance with the conventionally known method of producing a rapidly disintegrating tablet in an oral cavity after (a) a granule which contains a drug having a bitter taste and an excipient and in which the bitter taste has not been suppressed and (b) a granule containing a water soluble saccharide are appropriately mixed by a method usually used in the field of pharmaceutical preparation. For example, a method described in the above-mentioned Patent Documents 1 to 6 described in the Background Art may be employed.

In the production method of a rapidly disintegrating tablet in an oral cavity according to the present invention, the conventional mixing method in the field of pharmaceutical preparation may be applied in the following steps:

(i) a step of mixing (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent;

(ii) a step of mixing a lubricant with (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent [(c) a cool flavoring ingredient and a sweetener have been blended to one or both of (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent]; and

(iii) a step of mixing (c) a cool flavoring ingredient, a sweetener and a lubricant with (a) a granule which contains a drug having a bitter taste and an excipient and in which the bitter taste has not been suppressed, (b) a granule containing a water soluble saccharide which is insoluble in an alcohol based solvent and a binder which is soluble in water and in an alcohol based solvent. For example, a double cone blender (for example, Double cone mixer; produced by Yashima Chemical engineering Co., Ltd.), a fluidized bed granulator (for example, Multiplex; produced by Powrex, Co., Ltd., Spiraflow; produced by Freund Corporation.), a high speed agitating granulator (for example, High speed mixer; produced by Fukae Powtec, Co., Ltd., Vertical Granulator; produced by Powrex, Co., Ltd.) or a vibrating sieve (for example, Vibrating sieve; produced by Dalton, Co., Ltd.) and the like may be employed in the mixing method.

As for compression-molding of the above-mentioned mixture, it can be performed by utilizing the conventional tabletting machine such as Rotary three layers tabletting machine RT-3L 14; produced by Kikusui Seisakusho Ltd., Duplex tabletting Collect D65RC; produced by Kikusui Seisakusho Ltd., Clean Press Collect 18HUK; produced by Kikusui Seisakusho Ltd. For example, it can be carried out by the following methods:

(1) Compression-molding of the mixture containing a lubricant and a free-flowing agent and compression-molding of the mixture containing (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent, are alternately performed (Japanese Patent Application Laid-Open (JP-A) No. Hei 10-298061)

(2) Compression-molding of the mixture containing (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent, is performed while spraying a powder of a lubricant to the mixture (Japanese Patent Publication Nos. Sho 41-1273 and Sho 48-20103)

(3) The mixture containing (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent, is chamfered and molded via a fixation prevention film (JP-A No. Hei 08-19589), or

(4) A small amount of a lubricant which does not have an influence upon the disintegration property/dissolution property of a compression-molded pharmaceutical preparation is added to the mixture containing (a) a granule which contains a drug having a bitter taste and an excipient and in which the bitter taste has not been suppressed, and (b) a granule containing a water soluble saccharide which is insoluble in an alcohol based solvent and a binder which is soluble in water and in an alcohol based solvent, and the resulting mixture is molded.

The compression-molding may be performed by applying the molding pressure in the range of 5 to 60 MPa, preferably 15 to 40 MPa. As the shape of the compression-molded product, a tablet form, elliptical spherical shape, spherical shape, square type and the like are included. Moreover, from the viewpoint of ease of administration, it is preferably compression-molded so that the volume of the compression-molded product is in the range of 0.02 to 1 mL/tablet, preferably 0.05 to 0.5 mL/tablet.

As an alcohol based solvent used for alcohol treatment of a compression-molded product, methanol, ethanol, n-propanol, i-propanol, n-butanol, 2-methoxyethanol (Methyl cellosolve; produced by Katayama Chemical Industries, Co., Ltd.) and the like can be used. It is preferable that a solvent whose boiling point at normal pressure is 85°C or lower, for example, methanol, ethanol, n-propanol, i-propanol and the like are used, and it is particularly preferable that ethanol is used.

The treatment of the compression-molded product by an alcohol based solvent can be carried out by (i) adding the alcohol based solvent in a liquid state to the compression-molded product, (ii) spraying the alcohol based solvent to the compression-molded product, or (iii) after the compression-molded product is wetted by the vapor of the alcohol based solvent and so on, and by distilling off the alcohol based solvent from the compression-molded product by the conventional method such as heating, pressure reduction, ventilation and the like.

When it is wetted by the vapor of the alcohol based solvent, considering the stability of the drug, it is preferable that the vapor temperature is 60°C or less, and particularly, 40°C or less being preferred.

Moreover, as for a binder which is soluble in water and in an alcohol based solvent, after it is first dissolved by a treatment using an alcohol based solvent, which is performed after the compression-molding, it is solidified. With this treatment, the hardness of the compression-molded pharmaceutical preparation can be enhanced by the treatment using an alcohol based solvent, and the molded pharmaceutical preparation having the high hardness can be obtained even if it is molded by controlling the pressure at the time of compression molding comparatively low. For example, even when the hardness before it is treated by an alcohol based solvent is in the range of 2 to 15N, the hardness can be enhanced to the level of 20 to 80N by the treatment using an alcohol based solvent.

Since the binder contained in the compression-molded pharmaceutical preparation is dissolved by the treatment using an alcohol based solvent, when the treatment time is made long, to a certain extent, the amount of the binder dissolved is increased, and the increase in the hardness becomes large in the next drying step. When the necessary hardness is not obtained or the disintegration time in an oral cavity becomes long, the necessary hardness and the disintegration time of the rapidly disintegrating tablet in an oral cavity can be achieved by suitably selecting the amount of the binder in the compression-molded product, the treatment temperature or the treatment time, the amount of the alcohol based solvent which is applied.

Moreover, in the treatment using an alcohol based solvent in the method of producing a rapidly disintegrating tablet in an oral cavity according to the present invention, the binder which is soluble in water and in an alcohol based solvent can be dissolved, but the water soluble saccharide which is insoluble in the alcohol based solvent is not dissolved at all. Therefore, the shrinking, distortion and the like of the compression-molded product are hardly generated in the treatment stage using the alcohol based solvent, and the uniform rapidly disintegrating tablet in an oral cavity can be efficiently produced.

Since thus-obtained rapidly disintegrating tablet in an oral cavity has a hardness of 20N or more, an easily taking-out packaging form applicable to ordinary tablets such as a press-through-package (PTP) may be used therefor, and such tablet is useful due to the absence of limitation on delivery, carrying and handling after taken-out.

The PTP packaged product may be prepared by loading with one tablet each of tablet cavities of a resin film sheet, which has cavities for containing tablets and by shielding the resin film sheet with a conventional cover sheet for PTP packaging.

The material for the resin film sheet may not be limited to the extent that it is substantially transparent and flexible so that the tablet in the cavity can be pushed out by the finger pressure. Examples of such materials include poly-propylene, poly(vinyl chloride), poly-(vinylidene chloride) and the like. When moisture resistance is necessary, moisture resistant PTP sheet film such as a poly(vinyl chloride) film coated with poly-(trifluoroethylene chloride) [Aclar®; Honeywell], 5-layered film, poly(vinyl chloride)/ poly(vinylidene chloride)/ polyethylene/ poly(vinylidene chloride)/ poly(vinyl chloride) [Sumilite® VSL-4610N; Sumitomo Bakelite Co., Ltd.], ethylene-norbornene copolymer film [Polybar®; Alcan] and the like may be preferably used. On the other hand, examples of the cover sheet for PTP packaging includes moisture resistant sheets which may be broken with a weak power such as thin aluminum sheet and the like.

The production of (a) a granule which contains a drug having a bitter taste and an excipient and in which the bitter taste has not been suppressed, and the production of (b) a granule containing a water soluble saccharide which is insoluble in an alcohol based solvent and a binder which is soluble in water and in an alcohol based solvent, both of which are used in the method according to the present invention, can be carried out by the following steps:

(Production step of a granule which contains a drug having a bitter taste and an excipient and in which the bitter taste has not been suppressed)

A granule which contains a drug having a bitter taste and an excipient and in which the bitter taste has not been suppressed can be prepared, by using a mixture containing a drug having a bitter taste and an excipient, by a known granulation method such as a wet granulation method, a dry granulation method, a layering granulation method, a heat-melting granulation method, an immersion granulation method, a spray-drying granulation method and the like.

When preparing a granule by a wet granulation method, for example, the following method can be used:

(1) A binder solution is added to a mixture containing a drug having a bitter taste and an excipient (hereinafter, referred to as a drug mixture), agitated by utilizing a high speed agitating granulator and the like and granulated (WO00/24379).

(2) After a binder solution is added to a drug mixture and kneaded, the kneaded mass was granulated and screened by using an extrude granulator.

(3) A binder solution is sprayed to a drug mixture under fluidized condition using a fluidized bed granulator, an agitating fluidized bed granulator and the like and granulated (WO94/8709).

As the solvent for dissolving a binder and the like, water, an aqueous alcohol such as an aqueous ethanol and the like can be used.

For preparation by a dry granulation method, a drug mixture is granulated by utilizing a roller compacter, a roll granulator or the like.

For preparing a granule by utilizing a layering granulation method, a drug mixture is added under spraying a binder solution to an inactive carrier which has been rolled by utilizing a centrifugal fluidization granulator and the like, and the drug mixture is attached on the carrier. As the inactive carrier, saccharides such as crystal white sugar, crystalline cellulose, crystal sodium chloride and the like, or crystal of an inorganic salt, spherical granulated material [for example, spherical granulated material made of crystalline cellulose (for example, CELPHERE® CP-203; produced by Asahi Kasei Corporation), spherical granulated material made of crystalline cellulose and lactose (for example, NONPAREIL-105; produced by Freund Corporation), spherical granulated material made of a purified white sugar (for example, NONPAREIL-103; produced by Freund Corporation), spherical granulated material made of a purified white sugar and a corn starch (for example, NONPAREIL-101; produced by Freund Corporation)] and the like can be used.

For preparing a granule by utilizing a heat-melting granulation method, it can be prepared by utilizing the following method:

(a) The drug mixture containing a substance to be melted by heating (a heat-melting substance) such as polyethylene glycol, fats and oils, wax and the like is granulated by agitating at the temperature at which the heat-melting substance is melted by utilizing an agitating granulator, a high speed agitating granulator or the like.

(b) A drug mixture containing a heat-melting substance is added to an inactive carrier which has been rolled at the temperature at which the heat-melting substance is melted by utilizing a centrifugal fluidization granulator or the like and the drug mixture is attached on the carrier.

For preparing a granule by utilizing an immersion granulation method, a solution containing a drug at a suitable concentration and a porous carrier which is an excipient are mixed, and after the drug solution is sufficiently kept in the porous portion of the carrier, it is dried and the solvent is removed. As a porous carrier, which is an excipient, magnesium metasilicate aluminate (Neusilin; produced by Fuji Chemical Industries, Co., Ltd.), calcium silicate (Flowlight; produced by Eisai, Co., Ltd.) and the like, can be used.

For preparing a granule by utilizing a spray-drying granulation method, a solution or suspension of a drug mixture is sprayed in an air flow at a high temperature and the product is dried by utilizing a spray-drying machine such as a spray-dryer and the like.

When a cool flavoring ingredient, a sweetener and the like are contained in a granule which contains a drug having a bitter taste and an excipient and in which the bitter taste has not been suppressed, it can be prepared similarly by utilizing a known granulation method by adding these components to a drug mixture.

At this time, a cool flavoring ingredient and an excipient may be added in a form of a powder, or in a form in which these components have been preblended.

(Production step in which a granule containing a water soluble saccharide which is insoluble in an alcohol based solvent and a binder which is soluble in water and in an alcohol based solvent)

A granule containing a water soluble saccharide which is insoluble in an alcohol based solvent and a binder which is soluble in water and in an alcohol based solvent can be prepared by utilizing a known granulation method such as a wetting type granulation method, a drying type granulation method, a layering granulation method, a heat-melting granulation method, an immersing granulation method, a spray-drying granulation method and the like after a variety of additives are added, if necessary, as in the case of a granule which contains a drug having a bitter taste and an excipient and in which the bitter taste has not been suppressed.

As additives, in addition to a water soluble saccharide which is insoluble in an alcohol based solvent and a binder which is soluble in water and in an alcohol based solvent, the organic excipient, the inorganic excipient, the lubricant, the cool flavoring ingredient and the sweetener which are mentioned above, and the like, can be used. A binder which is soluble in water and in an alcohol based solvent may be added to a water soluble saccharide which is insoluble in an alcohol based solvent in a form of a powder or in a form of a solution in a solvent, depending on the kinds of the granulation method. Moreover, a cool flavoring ingredient and an excipient may be added as a powder or in a form in which these components have been preblended.

When both of a granule which contains a drug having a bitter taste and an excipient and in which the bitter taste has not been suppressed, and a granule containing a water soluble saccharide which is insoluble in an alcohol based solvent and a binder which is soluble in water and in an alcohol based solvent, are produced, a wetting type granulation method using a high speed agitating granulator, a wetting type granulation method using a fluidized bed granulator, an immersing granulation method and a spray-drying granulation method may be preferably used in order to prepare a granule of particle diameter in the range of about 50 to 250 μm.

Example 1

(1) Preparation of granule containing active pharmaceutical ingredient (API)

80 parts by weight of bepotastine besilate and 20 parts by weight of crystalline cellulose (Avicel PH-101; produced by Asahi Kasei Corporation) were charged into a high speed agitating granulator (supermicro-high speed mixer; produced by Fukae Powtec Co., Ltd.) and premixed for one minute. 8.8 parts by weight of purified water was added while agitating the mixture at 50°C at 1000 rotations/min. After the mixture was granulated for 5 minutes, the product was dried until the temperature of the product became 45°C or higher by utilizing a fluidized bed granulator (MP-01/03; produced by Powrex, Co., Ltd.) to give a granule having the average particle diameter of 159 μm and containing bepotastine besilate.

(2) Preparation of granule without API

After 30 parts by weight of 5 w/w% aqueous solution of polyvinyl pyrrolidone (Colidon K30; produced by BASF, Co., Ltd.) was sprayed to 98.5 parts by weight of D-mannitol in a fluidized state by utilizing a fluidized bed granulator (GPCG-120; produced by Gratt, Co., Ltd.). The mixture was granulated and the product was dried to give a granule without API.

(3) Preparation of rapidly disintegrating tablet in an oral cavity

0.3 parts by weight of mentha oil (produced by Suzuki Menthol, Co., Ltd.), 2 parts by weight of 1-menthol (produced by Takasago International Corporation) which was melted, and 9.2 parts by weight of crystalline cellulose (Avicel PH-102; produced by Asahi Kasei Corporation) were mixed to give a flavor portion. To 11.5 parts by weight of flavor portion, 12.5 parts by weight of the granule containing API obtained in the above-mentioned (1), 271.8 parts by weight of the granule without API obtained in the above-mentioned (2), 3.0 parts by weight of aspartame (produced by Ajinomoto, Co., Inc.) and 1.5 parts by weight of magnesium stearate as a lubricant were mixed to give a mixture for tabletting.
The tabletting pressure was adjusted so that the hardness of the tablet became about 6 N by utilizing a rotary type tabletting machine (COLLECT 12HUK, pestle size: diameter of 10 mm; produced by Kikusui Seisakusho LTD.), and tabletting was performed so that the weight of one tablet is adjusted to about 300 mg. After the obtained tablet was left to stand for 16 hours in a desiccator which was filled with ethanol vapor at 30°C, ethanol was distilled off by leaving the tablet at 45°C for 3 hours using a tray type drying machine to give a rapidly disintegrating tablet in an oral cavity. When the hardness was measured in the diameter direction by utilizing Schleuniger tablet hardness tester, the hardness of the rapidly disintegrating tablet in an oral cavity was 49 N. Moreover, the time needed for one tablet placed in the oral cavity to dissolve naturally (without the tablet being chewed or without the tongue being moved vigorously) (disintegration time) was 18 seconds.
Thus-obtained rapidly disintegrating tablet in an oral cavity is loaded in each of the cavities of a film sheet [10 cavities per one sheet] made of poly(vinyl chloride) film coated with poly(trifluoroethylene chloride) [Aclar®; Honeywell] and the loaded film sheet is shielded with a thin aluminum sheet by heat-sealing to obtain PTP package loaded with rapidly disintegrating tablets in an oral cavity.

Example 2

(1) Preparation of granule containing API

42 parts by weight of bepotastine besilate and 54 parts by weight of D-mannitol (produced by Nikken Chemical and Synthetic Industry) were charged into a high speed agitating granulator (supermicro-high speed mixer; produced by Fukae Powtec Co., Ltd.) and premixed for one minute. 40 parts by weight of 10 w/w% aqueous solution of hydroxypropyl cellulose (HPC-SL; produced by Nippon Soda, Co., Ltd.) was added thereto and the mixture was granulated for about 2 minutes. After granulation, the product was dried until the temperature of the product became 45°C or more by utilizing a fluidized bed granulator (MP-01/03; produced by Powrex, Co., Ltd.) to give a granule having the average particle diameter of 141 μm and containing bepotastine besilate.

(2) Preparation of rapidly disintegrating tablet in an oral cavity

2 parts by weight of melted 1-menthol (produced by Takasago International Corporation), 0.3 part by weight of mentha oil (produced by Suzuki Menthol, Co., Ltd.), and 1.0 part by weight of hydrated silicon dioxide (Carplex; produced by Shionogi & Co., Ltd.) were mixed to give a flavor portion. To 3.3 parts by weight of flavor portion, 23.2 parts by weight of the granule containing API obtained in the above-mentioned (1), 269.3 parts by weight of the granule without API obtained in the above-mentioned Example 1(2), 3.0 parts by weight of aspartame (produced by Ajinomoto, Co., Inc.), and 1.5 parts by weight of magnesium stearate as a lubricant were mixed to give a mixture for tabletting. The tabletting pressure was adjusted so that the hardness of the tablet became about 6 N by utilizing a rotary type tabletting machine (COLLECT 12HUK, pestle size: diameter of 10 mm; produced by Kikusui Seisakusho LTD.), and tabletting was performed so that the weight of one tablet is adjusted to about 300 mg. After the obtained tablet was left to stand at 30°C for 16 hours in a desiccator which was filled with ethanol vapor, ethanol was distilled off by leaving the tablet at 45°C for 3 hours using a tray type drying machine to give a rapidly disintegrating tablet in an oral cavity. When the hardness was measured in the diameter direction by utilizing Schleuniger tablet hardness tester, the hardness of the rapidly disintegrating tablet in an oral cavity was 68 N. Moreover, the time needed for one tablet placed in the oral cavity to dissolve naturally (without the tablet being chewed or without the tongue being moved vigorously) (disintegration time) was 19 seconds.
Thus-obtained rapidly disintegrating tablet in an oral cavity is loaded in each of the cavities of a film sheet [10 cavities per one sheet] made of 5-layered film, poly(vinyl chloride)/ poly(vinylidene chloride)/ polyethylene/ poly(vinylidene chloride)/ poly(vinyl chloride) [Sumilite® VSL-4610N; Sumitomo Bakelite Co., Ltd.] and the loaded film sheet is shielded with a thin aluminum sheet by heat-sealing to obtain PTP package loaded with rapidly disintegrating tablets in an oral cavity.

Example 3

(1) Preparation of granule containing API

16.7 parts by weight of bepotastine besilate and 82.5 parts by weight of D-mannitol (produced by Nikken Chemical and Synthetic Industry) were charged into a high speed agitating granulator (supermicro-high speed mixer; produced by Fukae Powtec Co., Ltd.) and premixed for one minute. 8.3 parts by weight of 10 w/w% aqueous solution of hydroxypropyl cellulose (HPC-SL; produced by Nippon Soda, Co., Ltd.) was added thereto, and the mixture was granulated for about 3 minutes. After granulation, the product was dried until the temperature of the product becomes 45°C or higher by utilizing a fluidized bed granulator (MP-01/03; produced by Powrex, Co., Ltd.) to give a granule having the average particle diameter of 127 μm and containing bepotastine besilate.

(2) Preparation of rapidly disintegrating tablet in an oral cavity

2.3 parts by weight of melted 1-menthol (produced by Takasago International Corporation), 0.3 parts by weight of mentha oil (produced by Suzuki Menthol, Co., Ltd.), and 1.4 parts by weight of hydrated silicon dioxide (Carplex; produced by Shionogi & Co., Ltd.) were mixed to give a flavor portion. To 4.0 parts by weight of flavor portion, 60 parts by weight of the granule containing API obtained in the above-mentioned (1), 232.2 parts by weight of the granule without API obtained in the above-mentioned Example 1(2), 3.0 parts by weight of aspartame (produced by Ajinomoto, Co., Inc.), and 1.6 parts by weight of magnesium stearate as a lubricant were mixed to give a mixture for tabletting. The tabletting pressure was adjusted so that the hardness of the tablet becomes about 6 N by utilizing a rotary type tabletting machine (COLLECT 12HUK, pestle size: diameter of 10 mm; produced by Kikusui Seisakusho LTD.), and tabletting was performed so that the weight of one tablet is adjusted to about 300 mg. After the obtained tablet was left to stand at 30°C for 16 hours in a desiccator which was filled with ethanol vapor, ethanol was distilled off by leaving the tablet at 45°C for 2 hours using a tray type drying machine to give a rapidly disintegrating tablet in an oral cavity. When the hardness was measured in the diameter direction by utilizing Schleuniger tablet hardness tester, the hardness of the rapidly disintegrating tablet in an oral cavity was 57 N. Moreover, the time needed for one tablet placed in the oral cavity to dissolve naturally (without the tablet being chewed or without the tongue being moved vigorously) (disintegration time) was 24 seconds.
Thus-obtained rapidly disintegrating tablet in an oral cavity is loaded in each of the cavities of a film sheet [10 cavities per one sheet] made of poly(vinyl chloride) [Sumilite® VSS-1202; Sumitomo Bakelite Co., Ltd.] and the loaded film sheet is shielded with a thin aluminum sheet by heat-sealing to obtain PTP package loaded with rapidly disintegrating tablets in an oral cavity.

Comparative Example 1

(1) Preparation of granule

3.5 parts by weight of bepotastine besilate and 95 parts by weight of D-mannitol (produced by Nikken Chemical and Synthetic Industry) were charged into a fluidized bed granulator (MP-01/3; produced by Powrex Co., Ltd.) and 30 parts by weight of 5% aqueous solution of polyvinyl pyrrolidone (Coridon 30K; produced by BASF, Co., Ltd.) was sprayed at the temperature of the inlet air of 60°C for about 15 minutes, and the mixture was granulated. After granulation, the inlet air temperature within the fluidized bed granulator was set at 70°C, the product was dried until the temperature of the exhaust air became 45°C or higher to give a core particle having the average particle diameter of 125 μm and containing bepotastine besilate.

(2) Preparation of rapidly disintegrating tablet in an oral cavity

2.0 parts by weight of melted 1-menthol (produced by Takasago International Corporation), 0.3 parts by weight of mentha oil (produced by Suzuki Menthol, Co., Ltd.), and 9.2 parts by weight of crystalline cellulose (Avicel PH-102; produced by Asahi Kasei Corporation) were mixed to give a flavor portion. To 11.5 parts by weight of flavor portion, 274.3 parts by weight of the granule obtained in the above-mentioned (1), 3.0 parts by weight of aspartame (produced by Ajinomoto, Co., Inc.), and 1.5 parts by weight of magnesium stearate as a lubricant were mixed to give a mixture for tabletting.

The tabletting pressure was adjusted so that the hardness of the tablet becomes about 6 N by utilizing a rotary type tabletting machine (COLLECT 12HUK, pestle size: diameter of 10 mm; produced by Kikusui Seisakusho Ltd.), and tabletting was performed so that the weight of one tablet is adjusted to about 300 mg. After the obtained tablet was left to stand at 30°C for 16 hours in a desiccator which was filled with ethanol vapor, ethanol was distilled off by leaving the tablet at 45°C for 3 hours using a tray type drying machine to give a rapidly disintegrating tablet in an oral cavity. When the hardness was measured in the diameter direction by utilizing Schleuniger tablet hardness tester, the hardness of the rapidly disintegrating tablet in an oral cavity was 54 N. Moreover, the time needed for one tablet placed in the oral cavity to dissolve naturally (without the tablet being chewed or without the tongue being moved vigorously) (disintegration time) was 18 seconds.

Experimental Example 1 (Dissolution test)

The dissolution test was performed with 900 mL of purified water for the rapidly disintegrating tablet in an oral cavity obtained in Examples 1, 3 and Comparative Example 1 in accordance with the Japanese Pharmacopoeia Fourteenth Edition, dissolution test method, paddle method (number of paddle rotations; 50 rpm; 37°C). The concentration of bepotastine besilate in the dissolution solution was calculated by measuring the absorbance at 260 nm, and the dissolution ratio was calculated from the concentration and the amount of dissolved solution. The results are shown in Fig. 1. Among the preparations of Examples 1, 3 and Comparative Example 1, the difference of the drug dissolution rate in water was not found.

Experimental Example 2 (Examination on ease of administration)

The examination on the taste at the administration (examination on ease of administration) of the rapidly disintegrating tablets in an oral cavity obtained in Example 1 and Comparative Example 1, between which no difference was found in the drug dissolution rate in Experimental Example 1, was carried out.

Each of 6 test subjects kept in the mouth one rapidly disintegrating tablet in an oral cavity obtained in Example 1, and the tablet was disintegrated in the oral cavity. All of 6 test subjects did not feel any bitter taste. On the other hand, when each of the same 6 test subjects kept in the mouth one rapidly disintegrating tablet in the oral cavity obtained in Comparative Example 1, and the tablet was disintegrated in the oral cavity. Five test subjects felt the bitter taste and 3 test subjects out of these 5 subjects felt considerably bitter taste.

It should be noted that the component composition of the rapidly disintegrating tablet in an oral cavity of Example 1 and Comparative Example 1 are as indicated in Table 1.

Table 1

|  | Example 1 | Comparative Example 1 |
|---|---|---|
| Bepotastine besilate | 9.99 mg | 9.92 mg |
| Crystalline cellulose | 11.69 mg | 9.51 mg |
| Mannitol | 267.45 mg | 269.29 mg |
| Polyvinyl pyrrolidone | 4.07 mg | 4.25 mg |
| Menthol | 2.00 mg | 2.07 mg |
| Mentha oil | 0.30 mg | 0.31 mg |
| Aspartame | 3.00 mg | 3.10 mg |
| Magnesium stearate | 1.50 mg | 1.55 mg |
| Total | 300.00 mg | 300.00 mg |

**Claims**

1.  A method of suppressing a bitter taste of a drug, which comprises utilizing (a) a granule which contains a drug having a bitter taste and an excipient and in which the bitter taste has not been suppressed and (b) a granule containing a water soluble saccharide, when a rapidly disintegrating tablet in an oral cavity is produced.

2.  The method according to claim 1, comprising further formulating (c) a cool flavoring ingredient and a sweetener in one or both of (a) and (b).

3.  The method according to claim 1, comprising further formulating (c) a cool flavoring ingredient and a sweetener as a component in addition to (a) and (b).

4.  The method according to claim 2 or 3, wherein the granule which contains a drug having a bitter taste and an excipient and in which the bitter taste has not been suppressed, is contained in the range of 2 to 40 w/w% of the rapidly disintegrating tablet in an oral cavity (the drug having a bitter taste is contained in the range of 1 to 35 w/w% of the rapidly disintegrating tablet in an oral cavity), the granule containing a water soluble saccharide is contained in the range of 40 to 97 w/w% of the rapidly disintegrating tablet in an oral cavity (the water soluble saccharide is contained in the range of 20 to 96 w/w% of the rapidly disintegrating tablet in an oral cavity), the cool flavoring ingredient is contained in the range of 0.1 to 1 w/w% of the rapidly disintegrating tablet in an oral cavity, and the sweetener is contained in the range of 0.2 to 5 w/w% of the rapidly disintegrating tablet in an oral cavity.

5.  The method according to any one of claims 1 to 4, wherein the water soluble saccharide is endothermically soluble.

6.  The method according to any one of claims 2 to 4, wherein the water soluble saccharide is one or two or more kinds selected from mannitol, xylitol, erythritol, maltitol, sorbitol, lactose, sucrose, maltose and trehalose, the cool flavoring ingredient is one or two or more kinds selected from menthol, cinerole, camphor, mentha oil, mint oil (peppermint oil, spearmint oil), mint powder, orange oil, fennel oil, cinnamon oil, clove oil, turpentine oil and eucalyptus oil, and the sweetener is one or two or more kinds selected from saccharine, saccharine sodium, aspartame, acesulfame potassium, sucralose, stevia and sormatin.

7.  A method of producing a rapidly disintegrating tablet in an oral cavity comprising the following steps:

    (1) a step of mixing (a) a granule which contains a drug having a bitter taste and an excipient and in which the bitter taste has not been suppressed and (b) a granule containing a water soluble saccharide which is insoluble in an alcohol based solvent and a binder which is soluble in water and in an alcohol based solvent,
    (2) a step of compression-molding the mixture of(1), and
    (3) a step of treating the compression-molded product of (2) with an alcohol based solvent.

8.  The method according to claim 7, comprising further formulating (c) a cool flavoring ingredient and a sweetener to one or both of (a) and (b) in (1), and mixing a lubricant as a component in addition to (a) and (b) in (1).

9. The method according to claim 7, comprising further formulating (c) a cool flavoring ingredient, a sweetener and a lubricant as a component in addition to (a) and (b) in (1).

10. The method according to claim 7, wherein the average particle diameter of the granule which contains a drug having a bitter taste and an excipient and in which the bitter taste has not been suppressed is 50 to 250 $\mu$m, and the average particle diameter of the granule containing a water soluble saccharide which is insoluble in an alcohol based solvent and a binder which is soluble in water and in alcohol based solvent is 50 to 250 $\mu$m.

11. The method according to claim 8 or 9, wherein the granule which contains a drug having a bitter taste and an excipient and in which the bitter taste has not been suppressed is contained in the range of 2 to 40 w/w% of the rapidly disintegrating tablet in an oral cavity [the drug having a bitter taste is contained in the range of 1 to 35 w/w% of the rapidly disintegrating tablet in an oral cavity], the granule containing a water soluble saccharide which is insoluble in an alcohol based solvent and a binder which is soluble in water and in an alcohol based solvent is contained in the range of 40 to 97 w/w% of the rapidly disintegrating tablet in an oral cavity [the water soluble saccharide which is insoluble in an alcohol based solvent is contained in the range of 20 to 96 w/w% of the rapidly disintegrating tablet in an oral cavity, and the binder which is soluble in water and in alcohol based solvent is contained in the range of 0.5 to 10w/w% of the rapidly disintegrating tablet in an oral cavity], the cool flavoring ingredient is contained in the range of 0.1 to 1 w/w% of the rapidly disintegrating tablet in an oral cavity, the sweetener is contained in the range of 0.2 to 5 w/w% of the rapidly disintegrating tablet in an oral cavity, and the lubricant is contained in the range of 0.2 to 2 w/w% of the rapidly disintegrating tablet in an oral cavity.

12. The method according to any one of claims 7 to 11, wherein the water soluble saccharide which is insoluble in an alcohol based solvent is endothermically soluble and the binder which is soluble in water and in an alcohol based solvent is a polymer binder.

13. The method according to claim 8, 9 or 11, wherein the water soluble saccharide which is insoluble in an alcohol based solvent is one or two or more kinds selected from mannitol, xylitol, erythritol, maltitol, sorbitol, lactose, sucrose, maltose and trehalose, the binder which is soluble in water and in an alcohol based solvent is one or two or more kinds selected from polyvinyl pyrrolidone and hydroxypropyl celullose, the cool flavoring ingredient is one or two or more kinds selected from menthol, cinerole, camphor, mentha oil, mint oil (peppermint oil, spearmint oil), mint powder, orange oil, fennel oil, cinnamon oil, clove oil, turpentine oil and eucalyptus oil, the sweetener is one or two or more kinds selected from saccharine, saccharine sodium, aspartame, acesulfame potassium, sucralose, stevia and sormatin, and the lubricant is one or two or more selected from talc, light anhydrous silicic acid, hydrated silicon dioxide, alkali earth metal stearate, sucrose higher fatty acid ester and glycerin higher fatty acid ester

14. The method according to any one of claims 7 to 13, wherein the alcohol based solvent is ethanol.

15. The method according to any one of claims 7 to 14, wherein the hardness of the rapidly disintegrating tablet in an oral cavity in which a bitter taste has been suppressed is 20 to 80 N, the porosity is 20 to 50%, and the disintegration time in an oral cavity is within 60 seconds.

16. The method according to any one of claims 1 to 15, wherein the drug having a bitter taste is one or two or more kinds selected from antipyretic, analgesic and antiphlogistic drug; steroidal anti-inflammatory drug; antiulcer agent; coronary vasodilator; peripheral vasodilator; antibiotic drug; synthesized antibacterial drug; antiviral drug; antispastic drug; antitussive drug; expectorant; bronchodilator; cardiotonic; diuretic; muscle relaxant; brain metabolism improving drug; minor tranquilizer; major tranquilizer; beta-blocker; antiarrythmic drug; arthrifuge drug; anticoagulant drug; drug for migraine; antiepileptic drug; antiallergic drug; antiemetic drug; antihypertensive drug; drug for hyperlipidemia; sympathomimetic drug; Alzheimer dementia therapeutic drug; oral antitumor drug; alkaloidal narcotic drug; vitamin; drug for increased frequency of micturition; angiotensin conversion enzyme inhibition drug; and drug for erectile dysfunction.

17. A rapidly disintegrating tablet in an oral cavity containing the following three components:

    (1) a granule which contains bepotastine besilate and an excipient and in which a bitter taste has not been suppressed,
    (2) a granule containing a water soluble saccharide which is insoluble in an alcohol based solvent and a binder which is soluble in water and in an alcohol based solvent, and
(3) a cool flavoring ingredient, a sweetener and a lubricant.

18. The rapidly disintegrating tablet in an oral cavity according to claim 17, wherein the average diameter of the granule which contains bepotastine besilate and an excipient and in which a bitter taste has not been suppressed is 50 to 250 $\mu$m, and the average particle diameter of the granule containing a water soluble saccharide which is insoluble in an alcohol based solvent and a binder which is soluble in water and in alcohol based solvent is 50 to 250 $\mu$m.

19. The rapidly disintegrating tablet in an oral cavity according to claim 18, wherein the granule which contains bepotastine besilate and an excipient and in which a bitter taste has not been suppressed is contained in the range of 2 to 40 w/w% of the rapidly disintegrating tablet in an oral cavity [bepotastine besilate is contained in the range of 1 to 35 w/w% of the rapidly disintegrating tablet in an oral cavity], the granule containing a water soluble saccharide which is insoluble in an alcohol based solvent and a binder which is soluble in water and in an alcohol based solvent is contained in the range of 40 to 97 w/w% of the rapidly disintegrating tablet in an oral cavity [the water soluble saccharide which is insoluble in an alcohol based solvent is contained in the range of 20 to 96 w/w% of the rapidly disintegrating tablet in an oral cavity, and the binder which is soluble in water and in an alcohol based solvent is contained in the range of 0.5 to 10 w/w% of the rapidly disintegrating tablet in an oral cavity], the cool flavoring ingredient is contained in the range of 0.1 to 1 w/w% of the rapidly disintegrating tablet in an oral cavity, the sweetener is contained in the range of 0.2 to 5 w/w% of the rapidly disintegrating tablet in an oral cavity, and the lubricant is contained in the range of 0.2 to 2 w/w% of the rapidly disintegrating tablet in an oral cavity.

20. The rapidly disintegrating tablet in an oral cavity according to claim 19, wherein the hardness of the rapidly disintegrating tablet in an oral cavity is 20 to 80 N, the porosity is 20 to 50%, and the disintegration time in an oral cavity is within 60 seconds.

21. The rapidly disintegrating tablet in an oral cavity according to claim 20, wherein the water soluble saccharide which is insoluble in an alcohol based solvent is one or two or more kinds selected from mannitol, xylitol, erythritol, maltitol, sorbitol, lactose, sucrose, maltose and trehalose, the binder which is soluble in water and in an alcohol based solvent is one or two or more kinds selected from polyvinyl pyrrolidone and hydroxypropyl celullose, the cool flavoring ingredient is one or two or more kinds selected from menthol, cineole, camphor, mentha oil, mint oil (peppermint oil, spearmint oil), mint powder, orange oil, fennel oil, cinnamon oil, clove oil, turpentine oil and eucalyptus oil, the sweetener is one or two or more kinds selected from saccharine, saccharine sodium, aspartame, acesulfame potassium, sucralose, stevia and sormatin, and the lubricant is one or two or more selected from talc, light anhydrous silicic acid, hydrated silicon dioxide, alkali earth metal stearate, sucrose higher fatty acid ester and glycerin higher fatty acid ester.

22. The rapidly disintegrating tablet in an oral cavity according to claim 21, wherein the excipient is one or two kinds selected from mannitol and crystalline cellulose, the water soluble saccharide which is insoluble in an alcohol based solvent is mannitol, the binder which is soluble in water and in an alcohol based solvent is polyvinyl pyrrolidone, the cool flavoring ingredient is one or two or more kinds selected from menthol and mentha oil, the sweetener is aspartame, and the lubricant is one or two kinds selected from alkali earth metal stearates.

23. A method of producing a rapidly disintegrating tablet in an oral cavity comprising the following steps:

(1) a step of mixing (a) a granule which contains bepotastine besilate and an excipient and in which a bitter taste has not been suppressed, (b) a granule containing a water soluble saccharide which is insoluble in an alcohol based solvent and a binder which is soluble in water and in an alcohol based solvent and (c) a cool flavoring ingredient, a sweetener and a lubricant,
(2) a step of compression-molding the mixture of (1), and
(3) a step of treating the compression-molded product of (2) with the alcohol based solvent.

24. The method according to claim 23, wherein the alcohol based solvent is ethanol.

[Fig. 1]

Example 1 —○— Example 3 —□— Comparative Example 1 —●—

## INTERNATIONAL SEARCH REPORT

| International application No. |
| --- |
| PCT/JP2006/314467 |

A. CLASSIFICATION OF SUBJECT MATTER
*A61K9/20*(2006.01)i, *A61K31/4545*(2006.01)i, *A61K47/10*(2006.01)i, *A61K47/18* (2006.01)i, *A61K47/22*(2006.01)i, *A61K47/26*(2006.01)i, *A61K47/34*(2006.01)i, *A61K47/36*(2006.01)i, *A61K47/38*(2006.01)i, *A61K47/42*(2006.01)i,
According to International Patent Classification (IPC) or to both national classification and IPC

B. FIELDS SEARCHED

Minimum documentation searched (classification system followed by classification symbols)
A61K9/20, A61K31/4545, A61K47/10, A61K47/18, A61K47/22, A61K47/26, A61K47/34, A61K47/36, A61K47/38, A61K47/42, A61K47/44, A61K47/46, A61P37/08

Documentation searched other than minimum documentation to the extent that such documents are included in the fields searched
Jitsuyo Shinan Koho        1922-1996    Jitsuyo Shinan Toroku Koho    1996-2006
Kokai Jitsuyo Shinan Koho   1971-2006    Toroku Jitsuyo Shinan Koho    1994-2006

Electronic data base consulted during the international search (name of data base and, where practicable, search terms used)
WPI

C. DOCUMENTS CONSIDERED TO BE RELEVANT

| Category* | Citation of document, with indication, where appropriate, of the relevant passages | Relevant to claim No. |
| --- | --- | --- |
| X<br>A | JP 2003-034655 A (Takeda Chemical Industries, Ltd.),<br>07 February, 2003 (07.02.03),<br>All references; particularly, Par. Nos. [0015] to [0017]; examples 1 to 3<br>& WO 2002/092058 A1 | 1-6,16<br>7-15,17-24 |
| A | JP 2001-261553 A (Tanabe Seiyaku Co., Ltd.),<br>26 September, 2001 (26.09.01),<br>(Family: none) | 17-24 |
| A | JP 10-298061 A (Tanabe Seiyaku Co., Ltd.),<br>10 November, 1998 (10.11.98),<br>All references<br>& JP 3296412 B2 | 1-24 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 November, 2006 (01.11.06) | Date of mailing of the international search report<br>14 November, 2006 (14.11.06) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/314467

C (Continuation). DOCUMENTS CONSIDERED TO BE RELEVANT

| Category* | Citation of document, with indication, where appropriate, of the relevant passages | Relevant to claim No. |
|---|---|---|
| A | JP 8-291051 A (Sato Pharmaceutical Co., Ltd.), 05 November, 1996 (05.11.96), All references & JP 2919771 B2 | 1-24 |
| A | JP 5-271054 A (Takeda Chemical Industries, Ltd.), 19 October, 1993 (19.10.93), All references & JP 3069458 B2 & JP 2000-264836 A & US 5501861 A & US 5720974 A & EP 553777 A2 & EP 553777 A3 & EP 553777 B1 & CA 2088334 A & TW 249756 A & KR 258223 B1 & DE 69331839 E & CA 2088334 C | 1-24 |

Form PCT/ISA/210 (continuation of second sheet) (April 2005)

<table>
<tr><td>**INTERNATIONAL SEARCH REPORT**</td><td>International application No.<br>PCT/JP2006/314467</td></tr>
</table>

Continuation of A. CLASSIFICATION OF SUBJECT MATTER
(International Patent Classification (IPC))

*A61K47/44*(2006.01)i, *A61K47/46*(2006.01)i, *A61P37/08*(2006.01)i

(According to International Patent Classification (IPC) or to both national classification and IPC)

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI10298061 A
- JP HEI5271054 A
- JP HEI8291051 A
- WO 9315724 A
- WO 9520380 A
- JP HEI1112161 A
- WO 0202083 A
- JP 2000095707 A

- JP 2000159691 A
- JP 2001253818 A
- JP SHO411273 B
- JP SHO4820103 B
- JP HEI0819589 A
- WO 0024379 A
- WO 948709 A

**Non-patent literature cited in the description**

- Japanese Pharmacopoeia

- Double cone mixer. Yashima Chemical engineering Co., Ltd,